(19) Europäisches Patentamt
European Patent Office
Office européen des brevets

(11) **EP 1 960 398 B1**

(12) # EUROPEAN PATENT SPECIFICATION

(45) Date of publication and mention of the grant of the patent:
**29.09.2010 Bulletin 2010/39**

(21) Application number: **06819000.8**

(22) Date of filing: **07.12.2006**

(51) Int Cl.:
***C07D 471/04*** *(2006.01)* ***A61K 31/435*** *(2006.01)*
***A61P 29/00*** *(2006.01)*

(86) International application number:
**PCT/EP2006/011770**

(87) International publication number:
**WO 2007/065683 (14.06.2007 Gazette 2007/24)**

(54) **ARYL ACETIC ACID AND ESTER DERIVATIVES AND THEIR USES AS ANTIINFLAMMATORY**

ARYLESSIGSÄURE- UND ESTERDERIVATE UND DEREN VERWENDUNG ALS ENTZÜNDUNGSHEMMENDE MITTEL

DERIVES D ACIDES ET D ESTERS ARYL ACETIQUES AINSI QUE LEURS UTILISATIONS EN TANT QU AGENTS ANTIINFLAMMATOIRES

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.12.2005 GB 0525144**

(43) Date of publication of application:
**27.08.2008 Bulletin 2008/35**

(73) Proprietor: **Novartis AG**
**4056 Basel (CH)**

(72) Inventors:
- **BROWN, Lyndon Nigel**
**Horsham, West Sussex RH12 5AB (GB)**
- **LEBLANC, Catherine**
**Horsham, West Sussex RH12 5AB (GB)**
- **SANDHAM, David Andrew**
**Horsham, West Sussex RH12 5AB (GB)**
- **SINGH, Harinder Pal**
**Horsham, West Sussex RH12 5AB (GB)**

(74) Representative: **Bridle, Andrew Barry et al**
**Bridle Intellectual Property Ltd**
**6F Thomas Way**
**Lakeview International Business Park**
**Canterbury**
**Kent CT3 4JZ (GB)**

(56) References cited:
**WO-A2-2004/069782** **WO-A2-2005/123731**
**US-A- 3 320 268**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 960 398 B1

## Description

The present invention relates to organic compounds, their preparation and their use as pharmaceuticals.

WO 2004/069782 discloses the compound 1-isopropyl-2-methyl-1H-pyrrolo[2,3-b] pyridin-3-yl acetic acid used as an anti-inflammatory agent.

In a first aspect, the present invention provides compounds of formula **(I)**

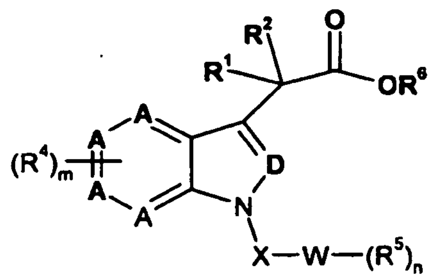

**(I)**

in free or salt form, wherein

$R^6$ is H, $C_1$-$C_8$-alkyl optionally substituted by $C_3$-$C_{15}$ carbocyclic group, ;
W is a $C_6$-$C_{15}$-aromatic carbocyclic group, a $C_3$-$C_{15}$-carbocyclic group, or a bond;
X is a bond, $C_2$-$C_6$-alkylene , or $C_1$-$C_8$-alkyleneoxy or, when W is phenyl, X is $C_2$-$C_8$ alkylene or $C_1$-$C_8$-alkyleneoxy;
wherein each $C_3$-$C_{15}$-carbocyclic group, $C_8$-$C_{15}$-membered aromatic carbocyclic group and each 4- to 14-membered heterocyclic group, unless otherwise specified is independently optionally substituted by one or more groups selected from halo, oxo, hydroxy, cyano, amino, nitro, carboxy, $C_1$-$C_8$-alkyl, halo-$C_1$-$C_8$-alkyl, $C_1$-$C_8$-alkoxy. $C_1$-$C_8$-alkylcarbonyl, $C_1$-$C_8$-alkylsulfonyl, $-SO_2NH_2$, ($C_1$-$C_8$-alkylamino)-sulfonyl, di($C_1$-$C_8$-alkyl)aminosulfonyl, aminocarbonyt, $C_1$-$C_8$-alkylaminocarbonyl and di($C_1$-$C_8$-alkyl)aminocarbonyl, a $C_3$-$C_{15}$-carbocyclic group, a $C_6$-$C_{15}$ membered carbocyclic group, a 4- to 14-membered heterocyclic group, cyano-$C_1$-$C_8$-alkyl, hydroxy-$C_1$-$C_8$-alkyl, $C_1$-$C_8$-haloalkyl, amino-$C_1$-$C_8$-alkyl, amino(hydroxy)$C_1$-$C_8$-alkyl and $C_1$-$C_8$-alkoxy optionally substituted by aminocarbonyl;
m and n are each, independently, an integer from 0-3;
the group

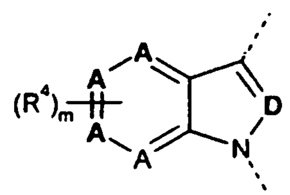

is

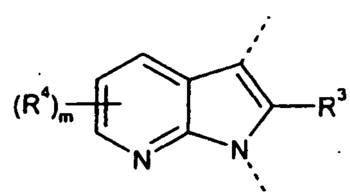

According to formula (I), $R^1$ and $R^2$ are suitably together H.

According to formula (I), $R^3$ is suitably $C_1$-$C_8$-alkyl, preferably methyl.

According to formula (I), $R^4$ is suitably not present

According to formula (I), $R^5$ is suitably not present or represents one or two groups independently selected from halogen, e.g. chloro, $C_1$-$C_8$-haloalkyl, e.g. trifluoromethyl, $C_1$-$C_8$-alkylsulfonyl, e.g. methylsulfonyl or ethylsulfonyl.

According to formula (I), $R^5$ is also suitably $-SO_2N(R^{5a})R^{5b}$, where $R^{5a}$ and $R^{5b}$ are suitably independently selected from $C_1$-$C_8$-alkyl, e.g. methyl and a $C_3$-$C_{15}$-carbocyclic group, e.g. cyclohexyl or $R^{5a}$ and $R^{5b}$ together with the

nitrogen atom to which they are attached suitably form a 4- to 14-membered heterocyclic group, e.g. 4-morpholinyl. More suitably, $R^{5a}$ and $R^{5b}$ together represent methyl and cyclohexyl or together form a 4-morpholinyl group.

According to formula (I), when W represents phenyl and $R^5$ is $C_1$-$C_8$-alkylsulfonyl, or-$SO_2N(R^{5a})R^{5b}$, $R^5$ is most preferably in the 4-phenyl position.

W is suitably a $C_6$-$C_{15}$-aromatic carbocyclic group, e.g. phenyl or a $C_3$-$C_{15}$- carbocyclic group, e.g.indanyl. Where W is phenyl, it is suitably unsubstituted, 4-subsituted or 2,4-substituted by $R^5$.

When W is phenyl, X is suitably $C_2$-$C_8$-alkylene, e.g. ethylene, 1-propylene, 2-propylene or 3-propylene, or $C_1$-$C_8$-alkyleneoxy, e.g. ethyleneoxy.

When W is indanyl (1, 2, or 3 indanyl), X is suitably a bond.

A preferred embodiment of the present invention provides compounds of formula (Ia)

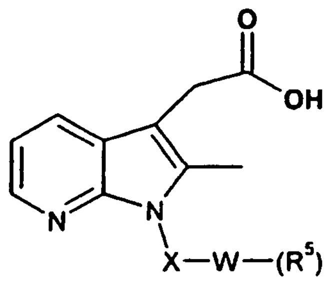

(Ia)

in free or salt form, where:

W represents phenyl and X represents $C_2$-$C_8$-alkylene or $C_1$-$C_8$-alkyleneoxy, or W represent indanyl and X represents a bond;

$R^5$ is not present or represents one or two groups independently selected from halogen, $C_1$-$C_8$-haloalkyl, $C_1$-$C_8$-alkylsulfonyl, -$SO_2N(R^{5a})R^{5b}$, where $R^{5a}$ and $R^{5b}$ are independently selected from $C_1$-$C_8$-alkyl and a $C_3$-$C_{15}$-carbocyclic group or $R^{5a}$ and $R^{5b}$ together with the nitrogen atom to which they are attached form a 4- to 14-membered heterocyclic group, e.g. 4-morpholinyl.

Terms used in the specification have the following meanings:

"Optionally substituted", as used herein, means the group referred to can be substituted at one or more positions by any one or any combination of the radicals listed thereafter.

"Halogen" or "halo" may be fluorine, chlorine, bromine or iodine.

"$C_1$-$C_8$-alkyl" denotes straight-chain or branched $C_1$-$C_8$-alkyl, which may be, e.g., methyl, ethyl, *n*-propyl, isopropyl, *n*-butyl, isobutyl, sec-butyl, *tert*-butyl, straight- or branched-pentyl, straight- or branched-hexyl, straight- or branched-heptyl or straight- or branched-octyl.

"$C_3$-$C_{15}$-carbocyclic group", as used herein, denotes a carbocyclic group having 3- to 15-ring carbon atoms that is saturated or partially saturated, such as a $C_3$-$C_8$-cycloalkyl. Examples of $C_3$-$C_{15}$-carbocyclic groups include but are not limited to cyclopropyl, cyclobutyl, cyclopentyl, cyclohexyl, cycloheptyl or cyclooctyl or a bicyclic group, such as bicyclooctyl, bicyclononyl including indanyl and indenyl, and bicyclodecyl.

"$C_6$-$C_{15}$-aromatic carbocyclic group", as used herein, denotes an aromatic group having 6-to 15-ring carbon atoms. Examples of $C_6$-$C_{15}$-Aromatic carbocyclic groups include but are not limited to phenyl, phenylene, benzenetriyl, naphthyl, naphthylene, naphthalenetriyl or anthrylene.

"$C_1$-$C_8$-alkoxy" denotes straight-chain or branched $C_1$-$C_8$-alkoxy which may be, e.g., methoxy, ethoxy, *n*-propoxy, isopropoxy, *n*-butoxy, isobutoxy, sec-butoxy, *tert*-butoxy, straight- or branched-pentoxy, straight- or branched-hexyloxy, straight- or branched-heptyloxy or straight- or branched-octyloxy. Preferably, $C_1$-$C_8$-alkoxy is $C_1$-$C_4$-alkoxy.

"$C_1$-$C_8$-haloalkyl" and "$C_1$-$C_8$-haloalkoxy" denote $C_1$-$C_8$-alkyl and $C_1$-$C_8$-alkoxy as hereinbefore defined substituted by one or more halogen atoms, preferably one, two or three halogen atoms, preferably fluorine, bromine or chlorine atoms. Preferably, $C_1$-$C_8$-haloalkyl is $C_1$-$C_4$-alkyl substituted by one, two or three fluorine, bromine or chlorine atoms.

"$C_1$-$C_8$-alkylsulfonyl", as used herein, denotes $C_1$-$C_8$-alkyl as hereinbefore defined linked to -$SO_2$-.

"$C_1$-$C_8$-alkylsulfinyl", as used herein, denotes $C_1$-$C_8$-alkyl as hereinbefore defined linked to -WO-.

"Amino-$C_1$-$C_8$-alkyl" and "amino-$C_1$-$C_8$-alkoxy" denote amino attached by a nitrogen atom to $C_1$-$C_8$-alkyl, e.g., $NH_2$-($C_1$-$C_8$)-, or to $C_1$-$C_8$-alkoxy, e.g., $NH_2$-($C_1$-$C_8$)-O-, respectively, as hereinbefore defined.

"Amino-(hydroxy)-$C_1$-$C_8$-alkyl" denotes amino attached by a nitrogen atom to $C_1$-$C_8$-alkyl and hydroxy attached by an oxygen atom to the same $C_1$-$C_8$-alkyl.

"Carboxy-$C_1$-$C_8$-alkyl" and "carboxy-$C_1$-$C_8$-alkoxy" denote carboxy attached by a carbon atom to $C_1$-$C_8$-alkyl or $C_1$-$C_8$-alkoxy, respectively, as hereinbefore defined.

"$C_1$-$C_8$-alkylcarbonyl", "$C_1$-$C_8$-alkoxycarbonyl "and "$C_1$-$C_8$-haloalkylcarbonyl" denote $C_1$-$C_8$-alkyl, $C_1$-$C_8$-alkoxy or $C_1$-$C_8$-haloalkyl, respectively, as hereinbefore defined attached by a carbon atom to a carbonyl group. "$C_1$-$C_8$-alkoxycarbonyl" denotes $C_1$-$C_8$-alkoxy as hereinbefore defined wherein the oxygen of the alkoxy group is attached to the carbonyl carbon.

"$C_1$-$C_8$-alkylamino" and "di($C_1$-$C_8$-alkyl)amino" denote $C_1$-$C_8$-alkyl as hereinbefore defined attached by a carbon atom to an amino group. The $C_1$-$C_8$-alkyl groups in di($C_1$-$C_8$-alkyl)amino may be the same or different.

"$C_1$-$C_8$-alkylaminocarbonyl" and "di($C_1$-$C_8$-alkyl)aminocarbonyl" denote $C_1$-$C_8$-alkylamino and di($C_1$-$C_8$-alkyl) amino, respectively, as hereinbefore defined attached by a nitrogen atom to the carbon atom of a carbonyl group.

"Di($C_1$-$C_8$-alkyl)amino-$C_1$-$C_8$-alkyl" and "di($C_1$-$C_8$-alkyl)amino-$C_1$-$C_8$-alkoxy" denote di($C_1$-$C_8$-alkyl)amino as hereinbefore defined attached by a nitrogen atom to the carbon atom of a $C_1$-$C_8$-alkyl or a $C_1$-$C_8$-alkoxy group, respectively.

"4- to 14-membered heterocyclic group ", refers to a 4- to 14-membered heterocyclic ring containing at least one ring heteroatom selected from the group consisting of nitrogen, oxygen and sulphur, which may be saturated, partially saturated or unsaturated (aromatic). Examples of 4- to 14- membered heterocyclic groups include but are not limited to furan, azetidine, pyrrole, pyrrolidine, pyrazole, imidazole, triazole, isotriazole, tetrazole, thiadiazole, isothiazole, oxadiazole, pyridine, piperidine, pyrazine, oxazole, isoxazole, pyrazine, pyridazine, pyrimidine, piperazine, pyrrolidine, pyrrolidinone, morpholine, triazine, oxazine, tetrahyrofuran, tetrahydrothiophene, tetrahydrothiopyran, tetrahydropyran, 1,4-dioxane, 1,4-oxathiane, indazole, quinoline, indazole, indole or thiazole.. The 4- to 14-membered heterocyclic group can be unsubstituted or substituted. Preferred substituents include halo, cyano, oxo, hydroxy, carboxy, nitro, $C_1$-$C_8$-alkyl, $C_1$-$C_8$-alkylcarbonyl, cyano-$C_1$-$C_8$-alkyl, hydroxy-$C_1$-$C_8$-alkyl, $C_1$-$C_8$-haloalkyl, amino-$C_1$-$C_8$-alkyl, amino(hydroxy) $C_1$-$C_8$-alkyl and $C_1$-$C_8$-alkoxy optionally substituted by aminocarbonyl. Especially preferred substituents include halo, oxo, $C_1$-$C_4$-alkyl, $C_1$-$C_4$-alkylcarbonyl, hydroxy-$C_1$-$C_4$-alkyl, $C_1$-$C_4$-haloalkyl, amino-$C_1$-$C_4$-alkyl and amino(hydroxy) $C_1$-$C_4$-alkyl.

Throughout this specification and in the claims that follow, unless the context requires otherwise, the word "comprise", or variations, such as "comprises" or "comprising", will be understood to imply the inclusion of a stated integer or step or group of integers or steps but not the exclusion of any other integer or step or group of integers or steps.

Where in formula (I), m or n are 2, the two substituents may be the same or different. Where m or n are 3, two or all of the substituents may be the same, or all three may be different.

In a yet further aspect, the present invention provides for the use of a compound of formula (I) in any of the aforementioned embodiments, in free or salt form, for the manufacture of a medicament for the treatment of an inflammatory or allergic condition, particularly an inflammatory or obstructive airways disease.

<u>Salts and Isomers</u>

Many of the compounds represented by formula (I) are capable of forming acid addition salts, particularly pharmaceutically acceptable acid addition salts. Pharmaceutically acceptable acid addition salts of the compound of formula (I) include those of inorganic acids, e.g., hydrohalic acids, such as hydrochloric acid or hydrobromic acid; nitric acid; sulphuric acid; phosphoric acid; and organic acids, e.g., aliphatic monocarboxylic acids, such as formic acid, acetic acid, diphenylacetic acid, triphenylacetic acid, caprylic acid, dichloroacetic acid, trifluoroacetic acid, hippuric acid, propionic acid and butyric acid; aliphatic hydroxy acids, such as lactic acid, citric acid, gluconic acid, mandelic acid, tartaric acid or malic acid; dicarboxylic acids, such as adipic acid, aspartic acid, fumaric acid, glutamic acid, maleic acid, malonic acid, sebacic acid or succinic acid; aromatic carboxylic acids, such as benzoic acid, p-chlorobenzoic acid, or nicotinic acid; aromatic hydroxy acids, such as o-hydroxybenzoic acid, p-hydroxybenzoic acid, 1-hydroxynaphthalene-2-carboxylic acid or 3-hydroxynaphthalene-2-carboxylic acid; and sulfonic acids, such as ethanesulfonic acid, ethane-1,2-disulfonic acid, 2-hydroxyethane-sulfonic acid, methanesulfonic acid, (+)-camphor-10-sulfonic acid, benzenesulfonic acid, naphthalene-2-sulfonic acid, naphthalene-1,5-disulfonic acid or p-toluenesulfonic acid. These salts may be prepared from compounds of formula (I) by known salt-forming procedures.

Compounds of formula (I) which contain acidic, e.g., carboxyl, groups, are also capable of forming salts with bases, in particular, pharmaceutically acceptable bases, such as those well-known in the art; suitable such salts include metal salts, particularly, alkali metal or alkaline earth metal salts, such as sodium, potassium, magnesium, calcium or zinc salts; or salts with ammonia or pharmaceutically acceptable organic amines or heterocyclic bases, such as

benethamine, arginine, benzathine, diethanolamine, ethanolamine, 4(2-hydroxy-ethyl)morpholine, 1-(2-hydroxyethyl) pyrrolidine, *N*-methyl glucamine, piperazine, triethanol-amine or tromethamine. These salts may be prepared from compounds of formula (I) by known salt-forming procedures

In those compounds where there is an asymmetric carbon atom or an axis of chirality the compounds exist in individual optically active isomeric forms or as mixtures thereof, e.g., as racemic or diastereomeric mixtures. The present invention embraces both individual optically active *R* and *S* isomers, as well as mixtures, e.g., racemic or diastereomeric mixtures, thereof.

Specific preferred compounds of formula (I) are described hereinafter in the Examples.

The invention also provides a process for the preparation of compounds of formula (I), in free or salt form, which comprises the steps of:

(i) (A) for the preparation of compounds of formula (I), wherein $R^6$ is H, cleaving the ester group -$COOR^6$ in a compound of formula (I),

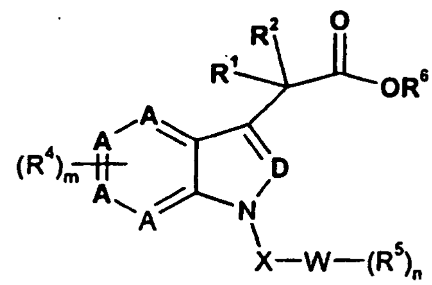

(I)

where $R^6$ is $C_1$-$C_8$-alkyl optionally substituted by $C_3$-$C_{15}$ carbocyclic group, or a $C_3$-$C_{15}$ carbocyclic group and $R^1$, $R^2$ $R^4$, $R^5$, A, D, W, X, m, and n are as hereinbefore defined; or

(B) for the preparation of compounds of formula (I), wherein $R^6$ is $C_1$-$C_8$-alkyl optionally substituted by $C_3$-$C_{15}$ carbocyclic group, or a $C_3$-$C_{15}$ carbocyclic group,
reacting a compound of formula (II)

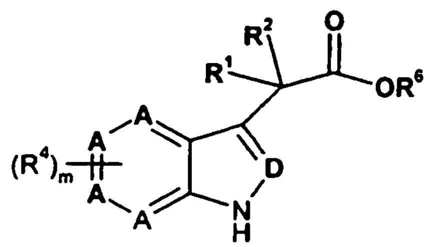

(II)

wherein
$R^6$ is $C_1$-$C_8$-alkyl optionally substituted by $C_3$-$C_{15}$ carbocyclic group, or a $C_3$-$C_{15}$ carbocyclic group, and
$R^1$, $R^2$, $R^4$, A. D, and m are as hereinbefore defined with a compound of formula (III)

G-X-W-$(R^5)_n$ (III)

wherein
G is a leaving moiety, e.g., a halogen atom;
$R^5$, W, X and n are as hereinbefore defined; and

(ii) recovering the resultant compound of formula (I) in free or salt form.

Process variant (A) may be carried out using known methods (or analogously as hereinafter described in the Examples) for cleavage of carboxylic ester groups and can be carried out *in situ* after preparation of a compound of formula (I), where $R^6$ is $C_1$-$C_8$-alky optionally substituted by $C_3$-$C_{15}$-carbocydic group, or a $C_3$-$C_{15}$-carbocyclic group, I. For example, the compound of formula (I), where $R^6$ is $C_1$-$C_8$-alkyl optionally substituted by $C_3$-$C_{15}$-carbocyclic group,

or a $C_3$-$C_{15}$-carbocyclic group, which is conveniently in solution in a polar organic solvent or a mixture thereof with water, may be reacted with an aqueous inorganic base, such as NaOH to hydrolyse the ester group; where the base is NaOH, the reaction may be carried out at a temperature of 10-40°C, conveniently ambient temperature.

Process variant (B) may be carried out using known procedures or analogously as hereinafter described in the Examples. For example, the compound of formula (II) may be reacted with an alkyl halide of formula (III), where
G is halogen; and
$R^5$, W, X and n are as hereinbefore defined,
in the presence of a base, such as NaH; the reaction may be carried out in an organic solvent, e.g., a polar aprotic solvent, such as *N*,*N*-dimethylformamide (DMF) and may be carried out at 10-40°C, conveniently at ambient temperature

Compounds of formula (II) are known or may be obtained by known methods, e.g., as described in U.S. Patent No. 3,320,268, or analogously as hereinafter described in the Examples. Compounds of formula (III) are known or may be obtained by known methods, or analogously, as hereinafter described in the Examples.

The compounds of formula (I) in free form may be converted into salt form, and vice versa, in a conventional manner. The compounds in free or salt form can be obtained in the form of hydrates or solvates containing a solvent used for crystallisation. Compounds of formulae (I) and (II) can be recovered from reaction mixtures and purified in a conventional manner. Isomers, such as enantiomers, may be obtained in a conventional manner, e.g., by fractional crystallisation, chiral HPLC resolution or asymmetric synthesis from correspondingly asymmetrically substituted, e.g., optically active, starting materials.

Pharmaceutical Use and Assay

Compounds of formula (I) and (II) and their pharmaceutically acceptable salts, hereinafter referred to alternatively as "agents of the invention", are useful as pharmaceuticals. In particular, the compounds have good CRTh2 receptor modulator activity and may be tested in the following assays.

Filtration binding assay protocol

The binding of CRTh2 modulators is determined using membranes prepared from human CRTh2-expressing Chinese Hamster Ovary cells (CHO.K1-CRTh2). To produce cell membrane CHO.K1-CRTh2 cells cultured in roller bottles are harvested using cell dissociation buffer (Invitrogen). The cells are pelleted by centrifugation (167 g, 5 min). The cell pellet is incubated in hypotonic buffer (15 mM Tris-OH, 2 mM $MgCl_2$, 0.3 mM EDTA, 1 mM EGTA, 1x Complete™ tablet) at 4°C for 30 min. At 4°C cells are homogenized using a Polytron® (IKA Ultra Turrax T25) for 5 bursts of 1 second. The homogenate is centrifuged (Beckman Optima TM TL Ultracentrifuge, 48000 g, 30 min at 4°C). The supernatant is discarded and the membrane pellet resuspended in homogenisation buffer (75 mM Tris-OH, 12.5 mM MgCl2, 0.3 mM EDTA, 1 mM EGTA, 250 mM Sucrose, 1x Complete™ tablet. Membrane preparations are aliquoted and stored at 80°C. The protein content is estimated using Bradford Protein Assay Dye (Bio Rad).

The binding of [$^3$H]-$PGD_2$ (157 Ci/mmol) to CHO.K1-CRTh2 membranes is determined in the absence (total binding) and presence (non-specific binding) of unlabelled $PGD_2$ (1 $\mu$M). Subtraction of the cpm (counts per minute) of [$^3$H]-$PGD_2$ binding in presence of excess unlabelled $PGD_2$ from that observed in the absence of excess unlabelled $PGD_2$ is defined as specific binding. Active CRTh2 modulators are able to compete with [$^3$H]-$PGD_2$ for binding to the CRTh2 receptor and are identified in a decrease in the number of cpm bound.

The assay is performed in Greiner U-bottomed 96 well-plates, in a final volume of 100 $\mu$l per well. CHO.K1-CRTh2 membranes were diluted in assay buffer (10mM HEPES-KOH (pH 7.4), 1 mM EDTA and 10 mM $MnCl_2$) and 10 $\mu$g are added to each well [$^3$H]-$PGD_2$ is diluted in assay buffer and added to each well at a final concentration of 2.5 nM. To determine non-specific binding, [$^3$H]-$PGD_2$ binding to the CRTh2 receptor is competed with using unlabelled $PGD_2$ at a final well concentration of 1 $\mu$M. The experiment is done in triplicate, with reagents added to the wells as follows:

- 25 $\mu$L assay buffer for total binding or
- 25 $\mu$L $PGD_2$ to determine non-specific binding
- 25 $\mu$L [$^3$H]$PGD_2$
- 50 $\mu$L membranes
- 25 $\mu$L test compound in DMSO/assay buffer

The plates are incubated at room temperature on a shaker for 1 hour, and then harvested (Tomtec Harvester

9600) onto GF/C filter plates using wash buffer (10 mM HEPES-KOH, pH 7.4). The plate is dried for 2 hours, prior to addition of Micro-Scint 20™ (50 μL) and sealing with TopSeal-S™. Plates are then counted using a Packard Top Count instrument, Plates are then read on the Packard Topcount with the 3H Scintillation program (1 min per well).

Ki (dissocation constant for the inhibition) values for the CRTh2 modulators are reported. Ki values are determined using Sigma Plot™ software, using the Cheng-Prussoff equation.

$$Ki = IC_{50} / 1 + [S]/Kd$$

where S is the concentration of radioligand and Kd is the dissociation constant.

CRTH2 cAMP functional assay protocol

This assay is conducted in CHO.K1-CRTh2 cells. cAMP is generated in the cell by stimulating cells with 5 μM forskolin, an adenylate cyclase activator. $PGD_2$ is added to activate the CRTh2 receptor which results in the attenuation of the forskolin-induced cAMP accumulation. Potential CRTh2 antagonists are tested for their ability to inhibit the $PGD_2$-mediated attenuation of the forskolin-induced cAMP accumulation in CHO.K1-CRTh2 cells.

For each concentration value on the dose-response curve, test compounds are prepared in assay stimulation buffer (HBSS, 5 mM HEPES, 10 μM IBMX ± 0.1% human serum albumin) containing DMSO (3% vol/vol) and 5 μL/well is added to an assay plate (384 well white optiplate).

CHO.K1-CRTh2 cultured in tissue culture flasks are washed with PBS and harvested with dissociation buffer. Cells are washed with PBS and resuspended in stimulation buffer to a concentration of $0.4 \times 10^8$/ mL and added to the assay plate (10 μL/well).

The assay plate is incubated at room temperature on a shaker for 15 minutes.

A mix of agonist (10 nM Prostaglandin $D_2$) and 5 μM forskolin is prepared in assay stimulation buffer and added to the assay plate (5 μL/well).

In addition, a cAMP standard is serially diluted in assay stimulation buffer and added to separate empty wells on the assay plate (20 μL/well). The cAMP standard allows for the quantification of cAMP generated in CHO.K1-CRTH2 cells.

The assay plate is incubated at room temperature on a shaker for 60 minutes.

Cell lysis buffer (Lysis buffer: Milli-Q $H_2O$, 5 mM HEPES, 0.3% Tween-20, 0.1 % human serum albumin) is added to a bead mix (containing Alphascreen™ anti-cAMP acceptor beads 0.06 units/μL, Alphascreen™ streptavidin-coated donor beads 0.06 units/μL, biotinylated cAMP 0.06 units/μL, 10μM IBMX) is prepared under darkened conditions 60 minutes prior to addition to the assay plate. The resulting lysis mix is added to all wells of the assay plate (40 μL/well).

The assay plate is sealed with Topseal-S™ and incubated in the dark at room temperature on a shaker for 45 minutes. The plate is then counted using a Packard Fusion™ instrument.

The resulting counts per minute are converted to nM cAMP by using the prepared cAMP standard curve. $IC_{50}$ values (concentration of CRTh2 antagonist required to inhibit 50% of the $PGD_2$-mediated attenuation of forskolin-induced cAMP accumulation in CHO.K1-CRTh2 cells) are then determined using Prism™ software.

Compound(s) of the Example(s) herein below generally have Ki values in the SPA binding assay below 1 μM. For example, the compound of Example 1 has a Ki value of 0.716 μM.

Compounds of formulae (I) and (II), in free or salt form, are modulators, of the G-protein-coupled chemoattractant receptor CRTh2, expressed on Th2 cells, eosinophils and basophils. $PGD_2$ is the natural ligand for CRTh2. Thus, modulators which inhibit the binding of CRTh2 and $PGD_2$ are useful in the treatment of allergic and anti-inflammatory conditions. Treatment in accordance with the invention may be symptomatic or prophylactic. "Modulators" as used herein is intended to encompass antagonists, agonists, partial antagonists and/or partial agonists. Preferably, modulators are antagonists.Accordingly, agents of the invention are useful in the treatment of inflammatory or obstructive airways diseases, resulting, e.g., in reduction of tissue damage, airways inflammation, bronchial hyperreactivity, remodelling or disease progression. Inflammatory or obstructive airways diseases to which the present invention is applicable include asthma of whatever type or genesis, including both intrinsic (non-allergic) asthma and extrinsic (allergic) asthma, mild asthma, moderate asthma, severe asthma, bronchitis asthma, exercise-induced asthma, occupational asthma and asthma induced following bacterial infection. Treatment of asthma is also to be understood as embracing treatment of subjects, e.g., of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g., of acute asthmatic or bronchoconstrictor attack, improvement in lung function or improved airways

hyperreactivity. It may further be evidenced by reduced requirement for other, symptomatic therapy, i.e., therapy for or intended to restrict or abort symptomatic attack when it occurs, e.g., anti-inflammatory (e.g., corticosteroid) or bronchodilatory. Prophylactic benefit in asthma may, in particular, be apparent in subjects prone to "morning dipping". "Morning dipping" is a recognised asthmatic syndrome, common to a substantial percentage of asthmatics and characterised by asthma attack, e.g., between the hours of about 4-6 a.m., i.e., at a time normally substantially distant from any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular, other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis including, e.g., aluminosis, anthracosis, asbestosis, chalicosis, ptilosis, siderosis, silicosis, tabacosis and byssinosis.

Having regard to their anti-inflammatory activity, in particular, in relation to inhibition of eosinophil activation, agents of the invention are also useful in the treatment of eosinophil related disorders, e.g., eosinophilia, in particular, eosinophils-related disorders of the airways, e.g., involving morbid eosinophilic infiltration of pulmonary tissues including hypereosinophilia as it effects the airways and/or lungs, as well as, e.g., eosinophil-related disorders of the airways consequential or concomitant to Löffler's syndrome; eosinophilic pneumonia; parasitic, in particular, metazoan, infestation including tropical eosinophilia; bronchopulmonary aspergillosis; polyarteritis nodosa including Churg-Strauss syndrome; eosinophilic granuloma; and eosinophil-related disorders affecting the airways occasioned by drug-reaction.

Agents of the invention are also useful in the treatment of inflammatory or allergic conditions of the skin, e.g., psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita and other inflammatory or allergic conditions of the skin.

Agents of the invention may also be used for the treatment of other diseases or conditions, in particular, diseases or conditions having an inflammatory component, e.g., treatment of diseases and conditions of the eye, such as conjunctivitis, keratoconjunctivitis sicca and vernal conjunctivitis; diseases affecting the nose including allergic rhinitis; and inflammatory disease, in which autoimmune reactions are implicated or having an autoimmune component or aetiology, including autoimmune hematological disorders, e.g., hemolytic anemia, aplastic anaemia, pure red cell anaemia and idiopathic thrombocytopenia; systemic lupus erythematosus; polychondritis; sclerodoma; Wegener granulamatosis; dermatomyositis; chronic active hepatitis; myasthenia gravis; Steven-Johnson syndrome; idiopathic sprue; autoimmune inflammatory bowel disease, e.g., ulcerative colitis and Crohn's disease; endocrine opthalmopathy; Grave's disease; sarcoidosis; alveolitis; chronic hypersensitivity pneumonitis; multiple sclerosis; primary billiary cirrhosis; uveitis (anterior and posterior); keratoconjunctivitis sicca and vernal keratoconjunctivitis; interstitial lung fibrosis; psoriatic arthritis; and glomerulonephritis, with and without nephrotic syndrome, e.g., including idiopathic nephrotic syndrome or minal change nephropathy.

Other diseases or conditions which may be treated with agents of the invention include septic shock; rheumatoid arthritis; osteoarthritis; proliferative diseases, such as cancer, mastocytosis, atherosclerosis; allograft rejection following transplantation; stroke; obesity; restenosis; diabetes, e.g., diabetes mellitus type I (juvenile diabetes) and diabetes mellitus type II; diarrhoeal diseases; ischemia/reperfusion injuries; retinopathy, such as diabetic retinopathy or hyperbaric oxygen-induced retinopathy; and conditions characterised by elevated intraocular pressure or secretion of ocular aqueous humor, such as glaucoma. Other diseases or conditions which may be treated with agents of the invention include neuropathic pain as described in WO 05/102338.

The effectiveness of an agent of the invention in inhibiting inflammatory conditions, e.g., in inflammatory airways diseases, may be demonstrated in an animal model, e.g., a mouse or rat model, of airways inflammation or other inflammatory conditions, e.g., as described by Szarka et al., J Immunol Methods, Vol. 202, pp. 49-57 (1997); Renzi et al., Am Rev Respir Dis, Vol. 148, pp. 932-939 (1993); Tsuyuki et al., J Clin Invest, Vol. 96, pp. 2924-2931 (1995); Cernadas et al., Am J Respir Cell Mol Biol, Vol. 20, pp. 1-8 (1999); and Williams and Galli, J Exp Med, Vol. 192, pp. 455-462 (2000).

The agents of the invention are also useful as co-therapeutic agents for use in combination with other drug substances, such as anti-inflammatory, bronchodilatory or antihistamine drug substances, particularly in the treatment of obstructive or inflammatory airways diseases, such as those mentioned hereinbefore, e.g., as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance. Accordingly the invention includes

a combination of an agent of the invention as hereinbefore described with an anti-inflammatory, bronchodilatory, antihistamine or anti-tussive drug substance, said agent of the invention and said drug substance being in the same or different pharmaceutical composition.

Such anti-inflammatory drugs include steroids, in particular, glucocorticosteroids, such as budesonide, beclamethasone dipropionate, fluticasone propionate, ciclesonide or mometasone furoate; or steroids, described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/035668, WO 03/048181, WO 03/062259, WO 03/064445 and WO 03/072592; non-steroidal glucocorticoid receptor agonists, such as those described in WO 00/00531, WO 02/10143, WO 03/082280, WO 03/082787, WO 03/104195 and WO 04/005229; LTB4 antagonists, such as those described in U.S. Patent No. 5,451,700; LTD4 antagonists, such as montelukast and zafirlukast; PDE4 inhibitors, such as cilomilast (Ariflo® GlaxoSmithKline), Roflumilast (Byk Gulden), V-11294A (Napp), BAY19-8004 (Bayer), SCH-351591 (Schering-Plough), Arofylline (Almirall Prodesfarma), PD189659 (Parke-Davis), AWD-12-281 (Asta Medica), CDC-801 (Celgene), SelCID(TM) CC-10004 (Celgene), KW-4490 (Kyowa Hakko Kogyo), WO 03/104204. WO 03/104205, WO 04/000814, WO 04/000839 and WO 04/005258 (Merck), as well as those described in WO 98/18796 and WO 03/39544; A2a agonists, such as those described in EP 1052264, EP 1241176, EP 409595A2, WO 94/17090, WO 96/02543, WO 96/02553, WO 98/28319, WO 99/24449, WO 99124450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462 and WO 03/086408; A2b antagonists, such as those described in WO 02/42298; and beta (β)-2-adrenoceptor agonists, such as albuterol (salbutamol), metaproterenol, terbutaline, salmeterol, fenoterol, procaterol, and especially, formoterol and pharmaceutically acceptable salts thereof, and compounds (in free or salt or solvate form) of formula (I) of WO 00/75114, preferably compounds of the Examples thereof, especially a compound of formula

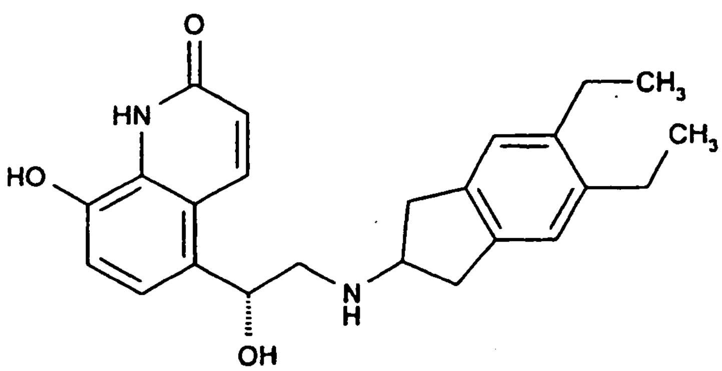

and pharmaceutically acceptable salts thereof, as well as compounds (in free or salt or solvate form) of formula (I) of WO 04/16601. Further β-2-adrenoreceptor agonists include compounds, such as those described in WO 99/64035, WO 01/42193, WO 01/83462, WO 02/066422, WO 02/070490, WO 02/076933, WO 2004/011416 and US 2002/0055651.

Such bronchodilatory drugs include anticholinergic or antimuscarinic agents, in particular, ipratropium bromide, oxitropium bromide, tiotropium salts and CHF 4226 (Chiesi), but also those described in WO 01/04118, WO 02/51841, WO 02/53564, WO 03/00840, WO 03/87094, WO 04/05285, WO 02/00652, WO 03/33495, WO 03/53966, EP 0424021, US 5171744 and US 3714357.

Such co-therapeutic antihistamine drug substances include cetirizine hydrochloride, acetaminophen, clemastine fumarate, promethazine, loratidine, desloratidine, diphenhydramine and fexofenadine hydrochloride.

Combinations of agents of the invention and steroids, β-2 agonists, PDE4 inhibitors or LTD4 antagonists may be used, e.g., in the treatment of COPD or, particularly, asthma. Combinations of agents of the invention and anticholinergic or antimuscarinic agents, PDE4 inhibitors, dopamine receptor agonists or LTB4 antagonists may be used, e.g., in the treatment of asthma or, particularly, COPD.

Other useful combinations of agents of the invention with anti-inflammatory drugs are those with antagonists of chemokine receptors, e.g., CCR-1, CCR-2, CCR-3, CCR-4, CCR-5, CCR-6, CCR-7, CCR-8, CCR-9, CCR-10, CXCR1, CXCR2, CXCR3, CXCR4 and CXCR5; particularly useful are CCR-3 antagonists, such as those described in WO 2002/026723, especially 4-{3-[(*S*)-4-(3,4-dichlorobenzyl)-morpholin-2-ylmethyl]-ureidomethyl}-benzamide and those described in WO 2003/077907, WO 2003/007939 and WO 2002/102775.

Also especially useful are CCR-5 antagonists, such as Schering-Plough antagonists SC-351125, SCH-55700 and SCH-D; Takeda antagonists, such as *N*-[[4-[[[6,7-dihydro-2-(4-methylphenyl)-5*H*-benzo-cyclohepten-8-yl]carbonyl] amino]phenyl]-methyl]tetrahydro-*N*,*N*-dimethyl-2*H*-pyran-4-aminium chlorine (TAK-770); and CCR-5 antagonists, described in US 6166037, WO 00/66558 and WO 00/66559.

The agents of the invention may be administered by any appropriate route, e.g.; orally, e.g., in the form of a tablet or capsule; parenterally, e.g., intravenously; by inhalation, e.g., in the treatment of inflammatory or obstructive airways disease: intranasally, e.g., in the treatment of allergic rhinitis; topically to the skin, e.g., in the treatment of atopic dermatitis; or rectally, e.g., in the treatment of inflammatory bowel disease.

The present invention also provides a pharmaceutical composition comprising a compound of formula (I) in free form or in the form of a pharmaceutically acceptable salt, optionally together with a pharmaceutically acceptable diluent or carrier therefore. The composition may contain a co-therapeutic agent, such as an anti-inflammatory, bronchodilatory or antihistamine drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g., patches. Compositions for inhalation may comprise aerosol or other atomizable formulations or dry powder formulations.

When the composition comprises an aerosol formulation, it preferably contains, e.g., a hydro-fluoro-alkane (HFA) propellant, such as HFA134a or HFA227 or a mixture of these, and may contain one or more co-solvents known in the art, such as ethanol (up to 20% by weight); and/or one or more surfactants, such as oleic acid or sorbitan trioleate; and/or one or more bulking agents, such as lactose. When the composition comprises a dry powder formulation, it preferably contains, e.g., the compound of formula (I) having a particle diameter up to 10 microns, optionally together with a diluent or carrier, such as lactose, of the desired particle size distribution and a compound that helps to protect against product performance deterioration due to moisture. When the composition comprises a nebulised formulation, it preferably contains, e.g., the compound of formula (I) either dissolved, or suspended, in a vehicle containing water, a co-solvent, such as ethanol or propylene glycol and a stabilizer, which may be a surfactant.

The invention includes:

(a) an agent of the invention in inhalable form, e.g., in an aerosol or other atomizable composition or in inhalable particulate, e.g., micronised form;
(b) an inhalable medicament comprising an agent of the invention in inhalable form;
(c) a pharmaceutical product comprising such an agent of the invention in inhalable form in association with an inhalation device; and
(d) an inhalation device containing an agent of the invention in inhalable form.

Dosages of agents of the invention employed in practising the present invention will of course vary depending, e.g., on the particular condition to be treated, the effect desired and the mode of administration. In general, suitable daily dosages for oral administration are of the order of 0.01-100 mg/kg.

The invention is illustrated by the following Examples.

## EXAMPLES

*General Conditions*

LCMS are recorded on an Agilent 1100 LC system with a Waters Xterra MS C18 4.6 x 100 5 μM column, eluting with 5-95% 10 mM aqueous ammonium bicarbonate in acetonitrile over 2.5 minutes, with negative ion electrospray ionization or 5-95% water +0.1% TFA in acetonitrile with positive ion electrospray ionization. $[M+H]^+$ refers to monoisotopic molecular weights.

*Abbreviations*

| | |
|---|---|
| $CH_2Cl_2$ | dichloromethane |
| $MgSO_4$ | magnesium sulfate |
| aq | aqueous |
| DMF | N,N-dimethylformamide |
| EtOAc | ethyl acetate |
| HCl | hydrochloric acid |
| MeCN | acetonitrile |
| HPLC | high performance liquid chromatography |

| | |
|---|---|
| NaH | sodium hydride |
| NaOH | sodium hydroxide |
| TFA | trifluoroacetic acid |
| $Et_3N$ | triethylamine |
| MeOH | methanol |
| PS-DIEA | polystyrene-diisopropylethylamine |

**Example 1**

**Preparation of (2-Methyl-1-phenethyl-1H-pyrrolo[2,3-b]pyridine-3-yl)-acetic acid:**

**a) (2-Methyl-1-phenethyl-1H-pyrrolo[2,3-b]pyridine-3-yl)-acetic acid methyl ester:**

To a stirring solution of (2-methyl-1H-indol-3-yl)-acetic acid methyl ester (0.1 g, 0.49 mmol) and NaI (0.073 g, 0.49 mmol) in dry DMF (2 ml) at room temperature, is added portionwise 95% NaH (0.012 g, 0.49 mmol). The reaction mixture is stirred at room temperature for 15 minutes. Then a solution of commercially available (2-bromo-ethyl)-benzene (0.91 g, 0.49 mmol) in dry DMF (1 ml) is shaken with PS-DIEA for 15 minutes and then added to the reaction mixture. The reaction mixture is stirred at 60 ˚C for 2 h. The reaction mixture is evaporated *in vacuo.* Purification by flash chromatography on IST isolute™ C18 cartridge affords a colourless oil. Further purification by flash chromatography on silica gel using 1:1 iso-hexane:EtOAc, gives the desired product; [M+H]+ 309.

To a stirring solution of (2-methyl-1-phenethyl-1H-pyrrolo[2,3-b]pyridine-3-yl)-acetic acid methyl ester (10 mg, 0.032 mmol) in MeOH (0.5 ml) at room temperature, is added 1N NaOH aq (0.5 ml). The reaction mixture is stirred at room temperature for 1.5 h. The reaction mixture is evaporated *in vacuo,* diluted with water and acidified to acidic pH with 2N HCl aq. $CH_2Cl_2$ is added and the organic phase is separated. Evaporation *in vacuo* affords the pure product as a white solid. [M+H]+ 295.

**Example 2**

Preparation of [2-Methyl-1-(3-phenyl-propyl)-1H-pyrrolo[2,3-b]pyridine-3-yl]-acetic acid:

The title compound is prepared analogously to Example 1 by replacing (2-bromo-ethyl)-benzene with (3-bromo-propyl) benzene to give [2-Methyl-1-(3-phenyl-propyl)-1H-pyrrolo[2,3-b]pyridine-3-yl]-acetic acid; [M+H]+ 309.

**Example 3**

**Preparation of [2-Methyl-1-(2-phenoxy-ethyl)-1H-pyrrolo[2,3-b]pyridine-3-yl]-acetic acid:**

The title compound is prepared analogously to Example 1 by replacing (2-bromo-ethyl)-benzene with (2-bromo-ethoxy)-benzene to give [2-Methyl-1-(2-phenoxy-ethyl)-1H-pyrrolo[2,3-b]pyridine-3-yl]-acetic acid PS. [M+H]+ 311.

**Example 7**

**Preparation of {1-[2-(4-Methanesulfonyl-phenyl)-2-oxo-ethyl-2-methyl-1H-pyrrolo[2,3-b]pyridine-3-yl]-acetic acid:**

**a) {1-[2-(4-Methanesulfonyl-phenyl)-2-oxo-ethyl]-2-methyl-1H-pyrrolo[2,3-b]pyridine-3-yl}-acetic acid methyl ester:**

To a stirring solution of (2-methyl-1 H-indol-3-yl)-acetic acid methyl ester (1.0 g, 4.9 mmol) and NaI (0.73 g, 4.9 mmol) in dry DMF (10 ml) at room temperature, is added portionwise 95% NaH (0.12 g, 4.9 mmol). The reaction mixture is stirred at room temperature for 15 minutes. Then a solution of commercially available 2-bromo-1-(4-methanesulfonyl-phenyl)-ethanone (1.36 g, 4.9 mmol) in dry DMF (5 ml) is shaken with PS-DIEA for 15 minutes and then added to the reaction mixture. The reaction mixture is stirred at 55 ˚C for 2 h. The reaction mixture is diluted with water (150ml) and extracted with dichloromethane (2 x 60ml). The organic extracts are combined and concentrated to dryness *in vacuo.* Trituration from ethyl acetate followed by the addition of iso-hexane is carried out to precipitate solid impurities which are filtered off. The filtrate is concentrated to dryness *in vacuo.* and recrystallised from methanol to give the desired product as a white solid; [M+H]+ 401.

**b) {1-[2-Hydroxy-2-(4-methanesulfonyl-phenyl)-ethyl]-2-methyl-1H-pyrrolo[2,3-b]pyridine-3-yl}-acetic acid methyl ester:**

To a stirring suspension of {1-[2-Hydroxy-2-(4-methanesulfonyl-phenyl)-ethyl]-2-methyl-1H-pyrrolo[2,3-b]pyridine-3-yl}-acetic acid methyl ester (60 mg, 0.15 mmol) in dry MeOH (4 ml) at 10 °C is added sodium borohydride (12 mg, 0.32 mmol). The reaction mixture is stirred at room temperature for 1 h. The reaction mixture is evaporated *in vacuo.* to remove methanol then quenched with water (10 ml) and extracted with dichloromethane (2 x 20ml). The organic extracts are combined and concentrated to dryness *in vacuo.* To give the desired product as a white foam; [M+H]+ 403.

**c) {1-[2-Iodo-2-(4-methanesulfonyl-phenyl)-ethyl]-2-methyl-1H-pyrrolo[2,3-b]pyridine-3-yl}-acetic acid methyl ester:**

To a stirring solution of triphenylphosphine (39 mg, 0.14 mmol) in dry dichloromethane (2 ml) at room temperature is added iodine (28 mg, 0.11 mmol). The reaction mixture is stirred at room temperature for 15 min. After this time, imidazole (11 mg, 0.16 mmol) is added followed by the addition of {1-[2-Hydroxy-2-(4-methanesulfonyl-phenyl)-ethyl]-2-methyl-1H-pyrrolo[2,3-b]pyridine-3-yl}-acetic acid methyl ester (40 mg, 0.10 mmol)as a solution in dry dichloromethane (1 ml). The reaction mixture is stirred at room temperature for 2 h. The reaction mixture is concentrated to dryness *in vacuo.* at room temperature then partitioned between dichloromethane/water (20 ml:10 ml). The organic phase is concentrated to dryness *in vacuo*. at room temperature to give the crude product; [M+H]+ 513.

**d) {1-[2-(4-Methanesulfonyl-phenyl)-2-oxo-ethyl]-2-methyl-1H-pyrrolo[2,3-b]pyridine-3-yl]-acetic acid methyl ester:**

To a stirring solution of {1-[2-Iodo-2-(4-methanesulfonyl-phenyl)-ethyl]-2-methyl-1H-pyrrolo[2,3-b]pyridine-3-yl}-acetic acid methyl ester (52 mg, 0.10 mmol) in dry DMSO (2 ml) is added at room temperature sodium borohydride (8 mg, 0.20 mmol). The reaction mixture is stirred at room temperature for 16 h. The reaction mixture is quenched with water (20 ml) and extracted with dichloromethane (2 x 40 ml). The organic phases are combined and evaporated *in vacuo.* Purification by flash chromatography on IST isolute™ C18 cartridge using 0.1% TFA in water / MeCN gives the desired product; [M+H]+ 387.

**e) {1-[2-(4-Methanesulfonyl-phenyl)-2-oxo-ethyl]-2-methyl-1H-pyrrolo[2,3-b]pyridine-3-yl]-acetic acid:**

To a stirring solution of {1-[2-(4-Methanesulfonyl-phenyl)-2-oxo-ethyl]-2-methyl-1H-pyrrolo[2,3-b]pyridine-3-yl}-acetic acid methyl ester (10 mg, 0.026 mmol) in MeOH (0.5 ml) at room temperature, is added 1N NaOH aq (0.5 ml). The reaction mixture is stirred at room temperature for 1 h. The reaction mixture is diluted with water (10 ml) and extracted with dichloromethane (20 ml). Purification by flash chromatography on IST isolute™ C18 cartridge using 0.1 % TFA in water / MeCN gives the desired product; [M+H]+ 373.

The following examples have been prepared using the process described herein. Intermediates are prepared by methods known to those skilled in the art.

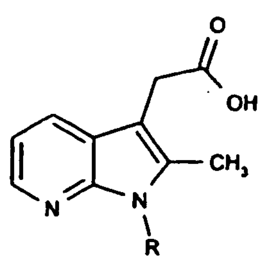

| Example | R |
|---|---|
| 1 | 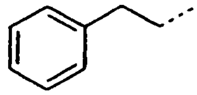 |
| 2 | 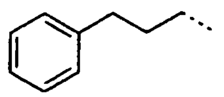 |
| 3 | 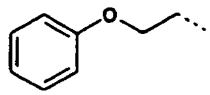 |
| 4 | 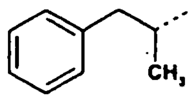 |
| 5 | 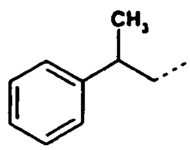 |
| 6 | 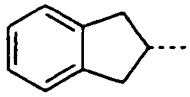 |
| 7 | 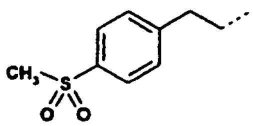 |
| 8 | 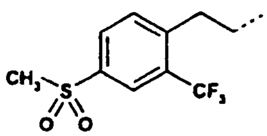 |
| 9 | 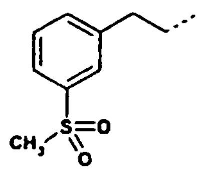 |
| 10 | 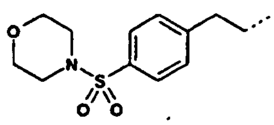 |
| 11 | 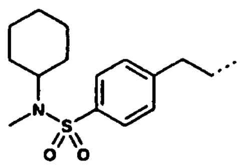 |

(continued)

| Example | R |
|---|---|
| 12 | 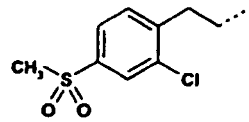 |
| 13 | 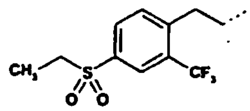 |
| 14 | 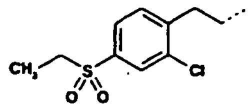 |
| 15 | 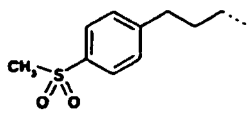 |
| 16 | 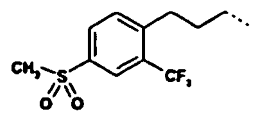 |
| 17 | 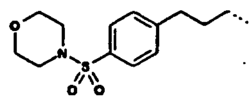 |
| 18 | 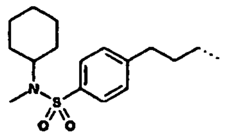 |
| 19 | 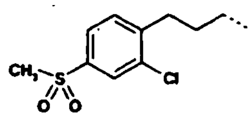 |
| 20 | 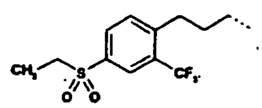 |
| 21 | 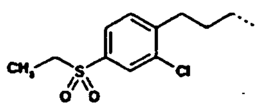 |
| 22 | 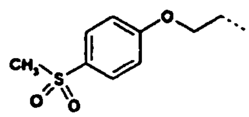 |
| 23 | 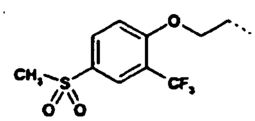 |

(continued)

| Example | R |
|---|---|
| 24 | 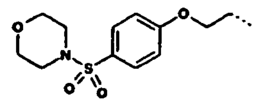 |
| 25 | 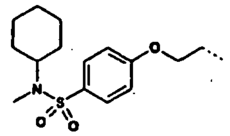 |
| 26 | 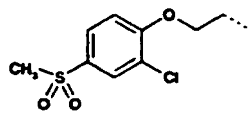 |
| 27 | 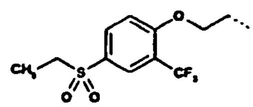 |
| 28 | 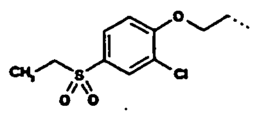 |
| 29 | 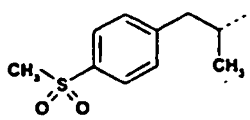 |
| 30 | 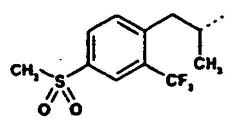 |
| 31 | 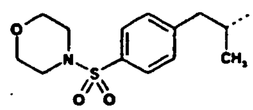 |
| 32 | 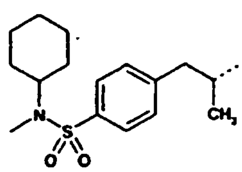 |
| 33 | 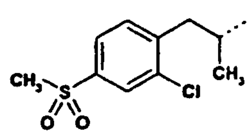 |
| 34 | 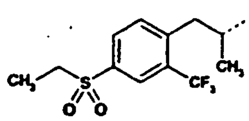 |
| 35 | 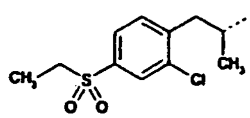 |

(continued)

| Example | R |
|---|---|
| 36 | 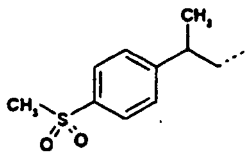 |
| 37 | 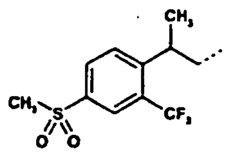 |
| 38 | 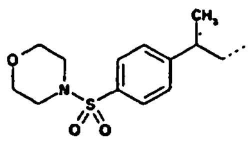 |
| 39 | 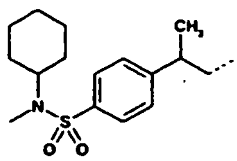 |
| 40 | 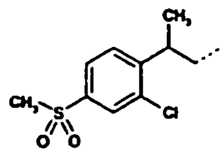 |
| 41 | 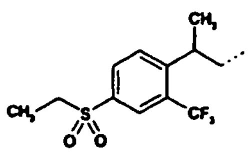 |
| 42 | 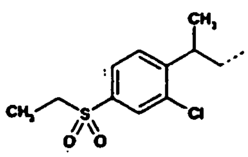 |
| 43 | 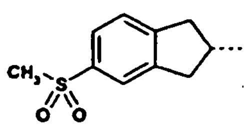 |

## Claims

**1.** A compound of formula (I)

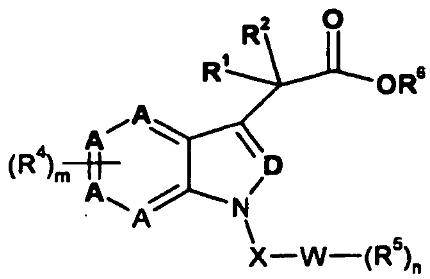

(I)

in free or salt form, wherein

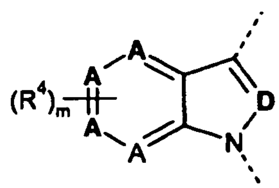

is

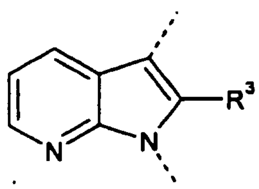

$R^1$ and $R^2$ are H;
$R^3$ is $C_1$-$C_8$-alkyl;
$R^5$ is one or two groups independently selected from halogen, $C_1$-$C_8$-haloalkyl, $C_1$-$C_8$-alkylsulfonyl or $R^5$ is $-SO_2N(R^{5a})R^{5b}$, where $R^{5a}$ and $R^{5b}$ are independently selected from $C_1$-$C_8$-alkyl, and a $C_3$-$C_{15}$-carbocyclic group, or $R^{5a}$ and $R^{5b}$ together with the nitrogen atom to which they are attached form a 4- to 14-membered heterocyclic group;
W is a bond, $C_6$-$C_{15}$-aromatic carbocyclic group, or a $C_3$-$C_{15}$- carbocyclic group;
X is a bond, $C_2$-$C_8$-alkylene or $C_1$-$C_8$-alkyleneoxy or, when W is phenyl, X is $C_2$-$C_8$-alkylene or $C_1$-$C_8$-alkyleneoxy;
n is 0-3;
$R^6$ is H or $C_1$-$C_8$-alkyl optionally substituted by a $C_3$-$C_{15}$ carbocyclic group; and wherein each $C_3$-$C_{15}$-carbocyclic group, $C_8$-$C_{15}$-membered aromatic carbocyclic group
and each 4- to 14-membered heterocyclic group, unless otherwise specified is independently optionally substituted by one or more groups selected from halo, oxo, hydroxy, cyano, amino, nitro, carboxy, $C_1$-$C_8$-alkyl, halo-$C_1$-$C_8$-alkyl, $C_1$-$C_8$-alkoxy, $C_1$-$C_8$-alkylcarbonyl, $C_1$-$C_8$-alkylsulfonyl, $-SO_2NH_2$, ($C_1$-$C_8$-alkylamino)-sulfonyl, di($C_1$-$C_8$-alkyl)aminosulfonyl, aminocarbonyl, $C_1$-$C_8$-alkylaminocarbonyl and di($C_1$-$C_8$-alkyl)aminocarbonyl, a $C_3$-$C_{15}$-carbocyclic group, a $C_6$-$C_{15}$-membered aromatic carbocyclic group, a 4- to 14-membered heterocyclic group, cyano-$C_1$-$C_8$-alkyl, hydroxy-$C_1$-$C_8$-alkyl, $C_1$-$C_8$-haloalkyl, amino-$C_1$-$C_8$-alkyl, amino(hydroxy)$C_1$-$C_8$-alkyl and $C_1$-$C_8$-alkoxy optionally substituted by aminocarbonyl.

**2.** A compound of formula (I) according to claim 1, wherein the compound is of formula (Ia)

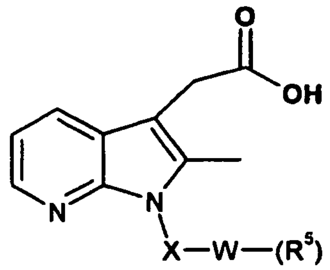

(Ia)

in free or salt form, where:

W is phenyl or indanyl;
X is a bond when W is indanyl and, when W is phenyl, X is $C_2$-$C_8$-alkylene or $C_1$-$C_8$-alkyleneoxy;
$R^5$ is not present or is one or two groups independently selected from halogen, $C_1$-$C_8$-haloalkyl, $C_1$-$C_8$-alkylsulfonyl, $-SO_2N(R^{5a})R^{5b}$, where $R^{5a}$ and $R^{5b}$ are independently selected from $C_1$-$C_8$-alkyl and a $C_3$-$C_{15}$-carbocyclic group or $R^{5a}$ and $R^{5b}$ together with the nitrogen atom to which they are attached form a 4- to 14-membered heterocyclic group.

**3.** A compound according to claim 1 or 2 having the formula

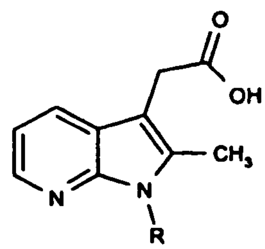

wherein R is

| Example | R |
|---|---|
| 1 | 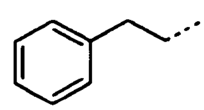 |
| 2 | 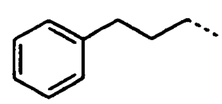 |
| 3 | 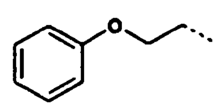 |
| 4 | 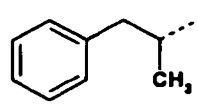 |

(continued)

| Example | R |
|---|---|
| 5 | 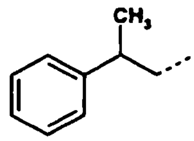 |
| 6 | 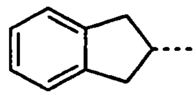 |
| 7 | 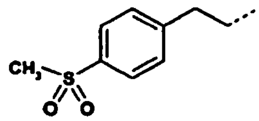 |
| 8 | 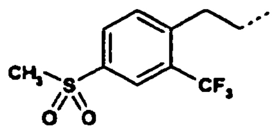 |
| 9 | 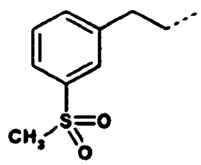 |
| 10 | 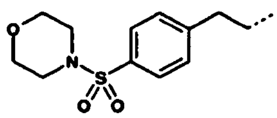 |
| 11 | 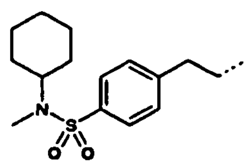 |
| 12 | 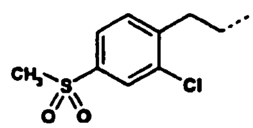 |
| 13 | 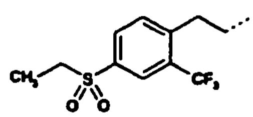 |
| 14 | 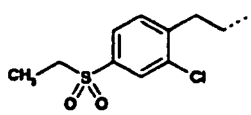 |
| 15 | 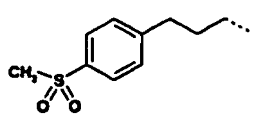 |

(continued)

| Example | R |
|---|---|
| 16 | 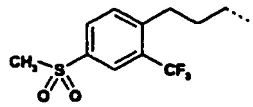 |
| 17 | 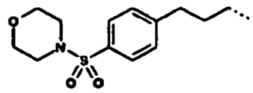 |
| 18 | 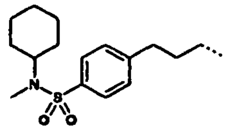 |
| 19 | 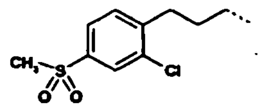 |
| 20 | 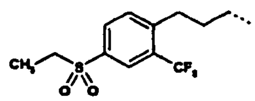 |
| 21 | 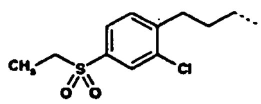 |
| 22 | 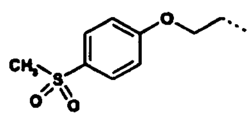 |
| 23 | 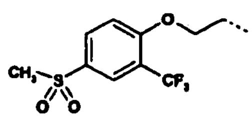 |
| 24 | 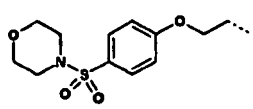 |
| 25 | 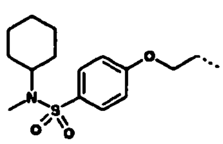 |
| 26 | 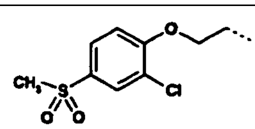 |
| 27 | 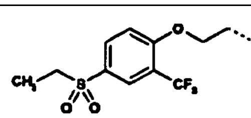 |

(continued)

| Example | R |
|---|---|
| 28 | 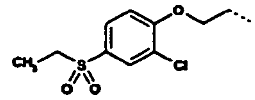 |
| 29 | 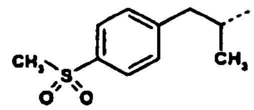 |
| 30 | 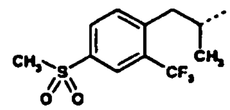 |
| 31 | 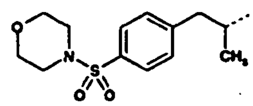 |
| 32 | 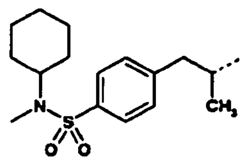 |
| 33 | 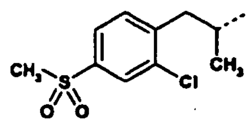 |
| 34 | 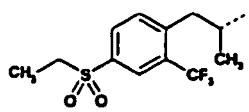 |
| 35 | 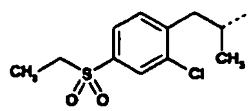 |
| 36 | 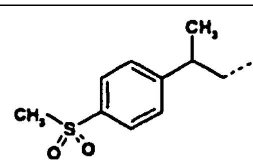 |
| 37 | 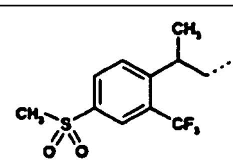 |
| 38 | 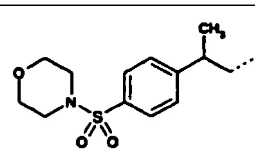 |

(continued)

| Example | R |
|---|---|
| 39 | 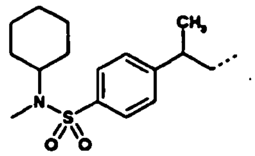 |
| 40 | 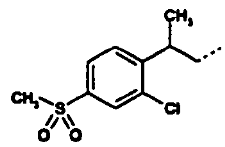 |
| 41 | 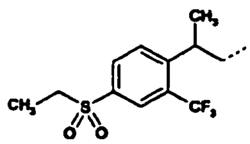 |
| 42 | 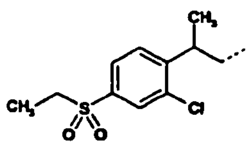 |
| 43 | 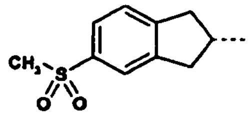 |

4. A compound according to any one of Claims 1 to 3 for use as a pharmaceutical.

5. Pharmaceutical compositions comprising a compound according to any one of Claims 1 to 3.

6. A compound according to any one of Claims 1 to 3 for treatment of neuropathic pain.

7. A compound according to any one of Claims 1 to 3 for treatment of an inflammatory or allergic condition, particularly an inflammatory or obstructive airways disease.

8. A process for the preparation of compounds of formula (I) as defined in Claim 1, in free or salt form, which comprises the steps of:

(i) (A) for the preparation of compounds of formula (I), wherein $R^6$ is H, cleaving the ester group -$COOR^8$ in a compound of formula (I),

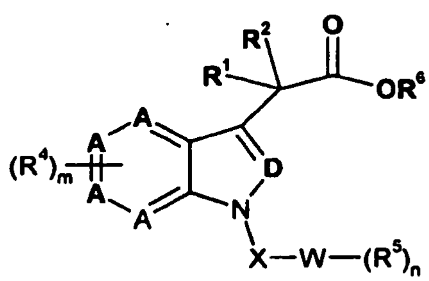

(I)

where $R^6$ is $C_1$-$C_8$-alkyl optionally substituted by a $C_3$-$C_{15}$ carbocyclic group and $R^1$, $R^2$, $R^4$, $R^5$, A, D, W, X,

m, and n are as hereinbefore defined; or

(B) for the preparation of compounds of formula (I), wherein $R^6$ is $C_1$-$C_8$-alkyl optionally substituted by a $C_3$-$C_{15}$ carbocyclic groupreacting a compound of formula (II)

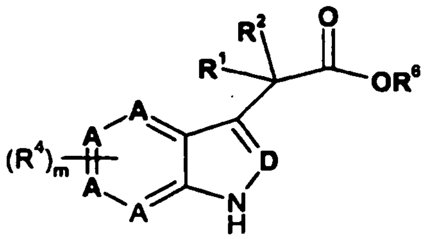

(II)

,

wherein

$R^6$ is $C_1$-$C_6$-alkyl optionally substituted by a $C_3$-$C_{15}$ carbocyclic group and $R^1$, $R^2$, $R^4$, A, D, and m are as hereinbefore defined with a compound of formula (III)

G-X-W-$(R^5)_n$ (III)

wherein

G is a leaving moiety;

$R^5$, W, X and n are as hereinbefore defined; and

(ii) recovering the resultant compound of formula (I) in free or salt form.

**Patentansprüche**

**1.** Verbindung mit der Formel (I)

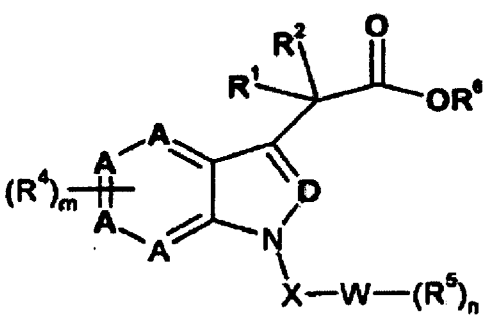

(I)

in freier oder salziger Form, wobei

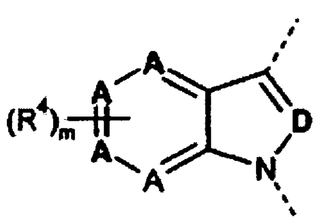

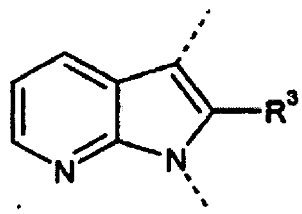

ist,
$R^1$ und $R^2$ H sind;
$R^3$ $C_1$-$C_8$-Alkyl ist;
$R^5$ eine oder zwei Gruppen sind, die unabhängig ausgewählt sind aus Halogen, $C_1$-$C_8$-Haloalkyl, $C_1$-$C_8$-Alkylsulfonyl, oder $R^5$-$SO_2N(R^{5a})R^{5b}$ ist, wobei $R^{5a}$ und $R^{5b}$ unabhängig ausgewählt sind aus $C_1$-$C_8$-Alkyl und einer $C_3$-$C_{15}$-carbocyclischen Gruppe, oder $R^{5a}$ und $R^{5b}$ zusammen mit dem Stickstoffatom, an das sie gebunden sind, eine 4- bis 14-gliedrige heterocyclische Gruppe bilden;
W eine Bindung, eine $C_6$-$C_{15}$-aromatische carbocyclische Gruppe oder eine $C_3$-$C_{15}$-carbocyclische Gruppe ist;
X eine Bindung, $C_2$-$C_8$-Alkylen oder $C_1$-$C_8$-Alkylenoxy oder, wenn W Phenyl ist, X $C_2$-$C_8$-Alkylen oder $C_1$-$C_8$-Alkylenoxy ist;
n 0-3 ist;
$R^6$ H oder $C_1$-$C_8$-Alkyl, wahlweise substituiert durch eine $C_3$-$C_{15}$-carbocyclische Gruppe ist; und
wobei jede $C_3$-$C_{15}$-carbocyclische Gruppe, $C_6$-$C_{15}$-gliedrige aromatische carbocyclische Gruppe und jede 4- bis 14-gliedrige heterocyclische Gruppe, sofern nicht anders angegeben, unabhängig wahlweise substituiert ist durch eine oder mehrere Gruppen, die ausgewählt sind aus Halo, Oxo, Hydroxy, Cyano, Amino, Nitro, Carboxy, $C_1$-$C_8$-Alkyl, Halo-$C_1$-$C_8$-Alkyl, $C_1$-$C_8$-Alkoxy, $C_1$-$C_8$-Alkylcarbonyl, $C_1$-$C_8$-Alkylsulfonyl, -$SO_2NH_2$, ($C_1$-$C_8$-Alkylamino)-Sulfonyl, Di($C_1$-$C_8$-Alkyl)aminosulfonyl, Aminocarbonyl, $C_1$-$C_8$-Alkylaminocarbonyl und Di($C_1$-$C_8$-Alkyl)aminocarbonyl, eine $C_3$-$C_{15}$-carbocyclische Gruppe, eine $C_6$-$C_{15}$-gliedrige aromatische carbocyclische Gruppe, eine 4- bis 14-gliedrige heterocyclische Gruppe, Cyano-$C_1$-$C_8$-Alkyl, Hydroxy-$C_1$-$C_8$-Alkyl, $C_1$-$C_8$-Haloalkyl, Amino-$C_1$-$C_8$-Alkyl, Amino(hydroxy)$C_1$-$C_8$-Alkyl und $C_1$-$C_8$-Alkoxy, wahlweise substituiert durch Aminocarbonyl.

**2.** Verbindung mit der Formel (I) gemäß Anspruch 1, wobei die Verbindung der Formel (Ia)

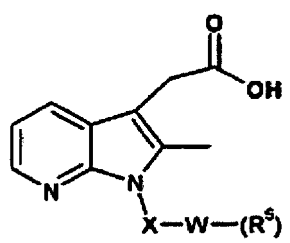

(Ia)

in freier oder salziger Form entspricht, wobei
W Phenyl oder Indanyl ist;
X eine Bindung ist, wenn W Indanyl ist, und wenn W Phenyl ist, X $C_2$-$C_8$-Alkylen oder $C_1$-$C_8$-Alkylenoxy ist;
$R^5$ nicht vorhanden ist oder eine oder zwei Gruppen sind, die unabhängig ausgewählt sind aus Halogen, $C_1$-$C_8$-Haloalkyl, $C_1$-$C_8$-Alkylsulfonyl, -$SO_2N(R^{5a})R^{5b}$ ist, wobei $R^{5a}$ und $R^{5b}$ unabhängig ausgewählt sind aus $C_1$-$C_8$-Alkyl und einer $C_3$-$C_{15}$-carbocyclischen Gruppe, oder $R^{5a}$ und $R^{5b}$ zusammen mit dem Stickstoffatom, an das sie gebunden sind, eine 4- bis 14-gliedrige heterocyclische Gruppe bilden.

**3.** Verbindung gemäß Anspruch 1 oder Anspruch 2 mit der Formel

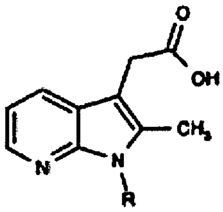

wobei R ist:

| Beispiel | R |
|---|---|
| 1 | 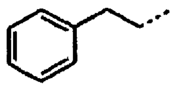 |
| 2 | 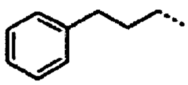 |
| 3 | 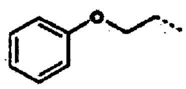 |
| 4 | 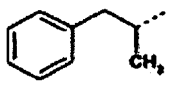 |
| 5 | 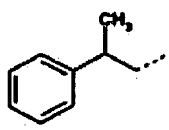 |
| 6 | 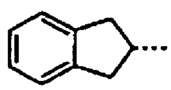 |
| 7 | 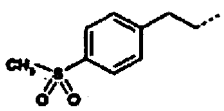 |
| 8 | 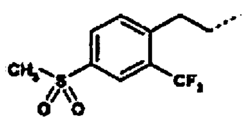 |
| 9 | 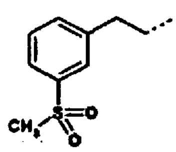 |
| 10 | 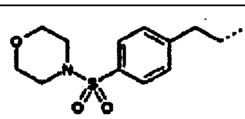 |

(fortgesetzt)

| Beispiel | R |
|---|---|
| 11 | 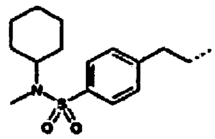 |
| 12 | 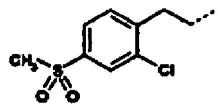 |
| 13 | 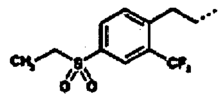 |
| 14 | 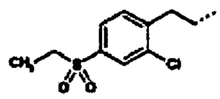 |
| 15 | 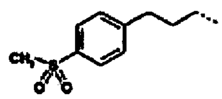 |
| 16 | 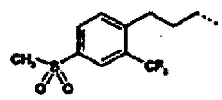 |
| 17 | 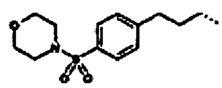 |
| 18 | 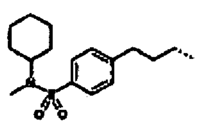 |
| 19 | 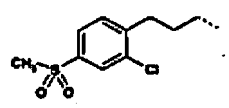 |
| 20 | 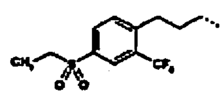 |
| 21 | 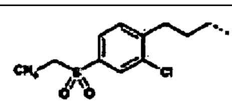 |
| 22 | 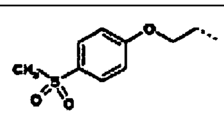 |
| 23 | 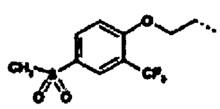 |
| 24 | 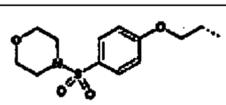 |

(fortgesetzt)

| Beispiel | R |
|---|---|
| 26 | 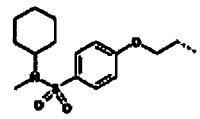 |
| 16 | 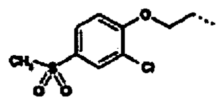 |
| 27 | 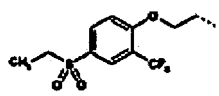 |
| 28 | 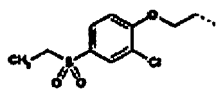 |
| 29 | 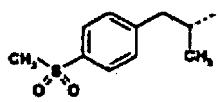 |
| 30 | 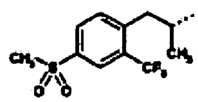 |
| 31 | 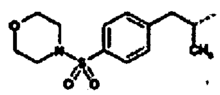 |
| 32 | 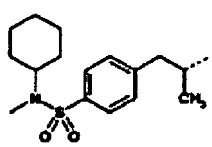 |
| 33 | 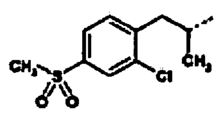 |
| 34 | 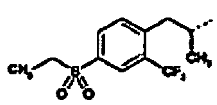 |
| 35 | 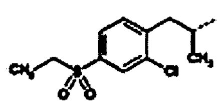 |
| 36 | 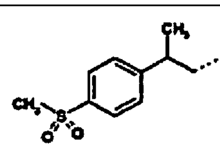 |
| 37 | 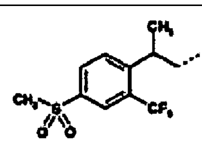 |

(fortgesetzt)

| Beispiel | R |
|---|---|
| 38 | 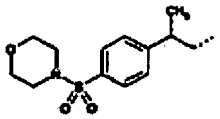 |
| 39 | 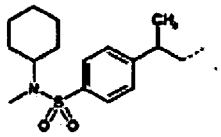 |
| 40 | 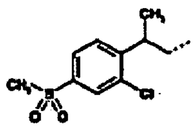 |
| 41 | 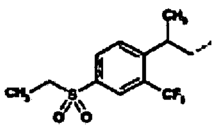 |
| 42 | 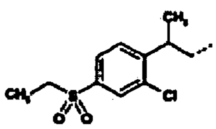 |
| 43 | 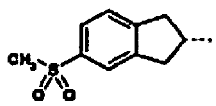 |

**4.** Zusammensetzung gemäß einem der Ansprüche 1 bis 3 zur Verwendung als Arzneimittel.

**5.** Pharmazeutische Zusammensetzungen, umfassend eine Verbindung gemäß einem der Ansprüche 1 bis 3.

**6.** Verbindung gemäß einem der Ansprüche 1 bis 3 zur Behandlung von neuropathischem Schmerz.

**7.** Verbindung gemäß einem der Ansprüche 1 bis 3 zur Behandlung eines inflammatorischen oder allergischen Zustands, insbesondere einer inflammatorischen oder obstruktiven Atemwegserkrankung.

**8.** Verfahren zur Herstellung von Verbindungen mit der Formel (I) gemäß Anspruch 1 in freier oder salziger Form, wobei das Verfahren die folgenden Schritte umfasst:

(i) (A) zur Herstellung von Verbindungen mit der Formel (I), wobei $R^6$ H ist, das Abspalten der Estergruppe $-COOR^6$ in einer Verbindung mit der Formel (I)

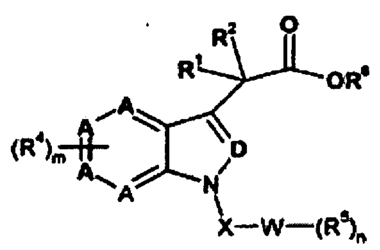

(I)

wobei $R^6$ $C_1$-$C_8$-Alkyl ist, wahlweise substituiert durch eine $C_3$-$C_{15}$-carbocyclische Gruppe, und $R^1$, $R^2$, $R^4$, $R^5$,

A, D, W, X, m und n wie zuvor definiert sind; oder
(B) zur Herstellung von Verbindungen mit der Formel (I), wobei $R^6$ $C_1$-$C_8$-Alkyl ist, wahlweise substituiert durch eine $C_3$-$C_{15}$-carbocydische Gruppe,
das Reagieren einer Verbindung mit der Formel (II)

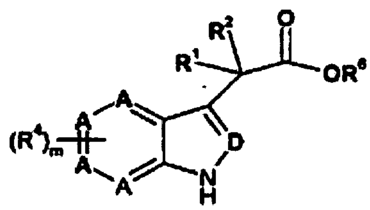

(II)

wobei
$R^6$ $C_1$-$C_8$-Alkyl ist, wahlweise substituiert durch eine $C_3$-$C_{15}$-carbocydische Gruppe und $R^1$, $R^2$, $R^4$, A, D und m wie zuvor definiert sind, mit einer Verbindung der Formel (III)

G-X-W-$(R^5)_n$ (III)

wobei
G eine abgehende Komponente ist;
$R^5$, W, X und n wie zuvor definiert sind; und
(ii) das Rückgewinnen der resultierenden Verbindung mit der Formel (I) in freier oder salziger Form.

**Revendications**

**1.** Un composé de formule (I)

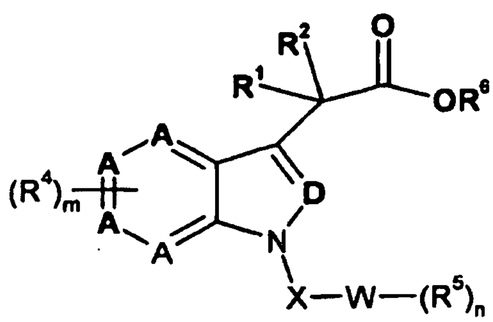

(I)

sous forme libre ou saline, où

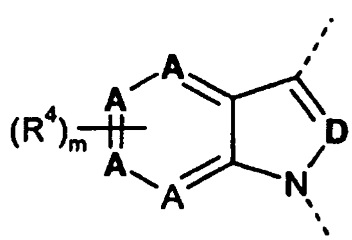

est

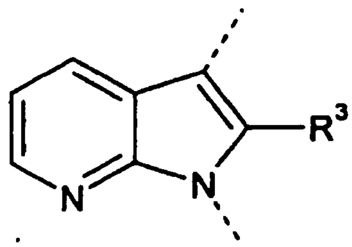

$R^1$ et $R^2$ sont H ;
$R^3$ est $C_1$-$C_8$-alkyle
$R^5$ est un ou deux groupe(s) indépendamment sélectionné(s) parmi halogène, $C_1$-$C_8$-haloalkyle, $C_1$-$C_8$-alkylsulfonyle ou $R^5$ est -$SO_2N(R^{5a})R^{5b}$, où $R^{5a}$ et $R^{5b}$ sont indépendamment sélectionnés parmi $C_1$-$C_8$-alkyle, et un groupe carbocyclique $C_3$-$C_{15}$, ou $R^{5a}$ et $R^{5b}$ ensemble avec l'atome d'azote auquel ils sont attachés forment un groupe hétérocyclique de 4 à 14 membres ;
W est une liaison, un groupe carbocyclique aromatique $C_6$-$C_{15}$, ou un groupe carbocyclique $C_3$-$C_{15}$ ;
X est une liaison, $C_2$-$C_8$-alkylène ou $C_1$-$C_8$-alkylèneoxy ou, quand W est phényle, X est $C_2$-$C_8$-alkylène ou $C_1$-$C_8$-alkylèneoxy ;
n est 0-3 ;
$R^6$ est H ou $C_1$-$C_8$-alkyle substitué de façon optionnelle par un groupe carbocyclique $C_3$-$C_{15}$ ; et
où chaque groupe carbocyclique $C_3$-$C_{15}$, groupe carbocyclique aromatique à membres $C_6$-$C_{15}$ et chaque groupe hétérocyclique de 4 à 14 membres, sauf spécification contraire, est indépendamment substitué de façon optionnelle par un ou plusieurs groupe(s) sélectionné(s) parmi halo, oxo, hydroxy, cyano, amino, nitro, carboxy, $C_1$-$C_8$-alkyle, halo-$C_1$-$C_8$-alkyle, $C_1$-$C_8$-alkoxy, $C_1$-$C_8$-alkylcarbonyle, $C_1$-$C_8$-alkylsulfonyle, -$SO_2NH_2$, ($C_1$-$C_8$-alkylamino)-sulfonyle, di($C_1$-$C_8$-alkyl)aminosulfonyle, aminocarbonyle, $C_1$-$C_8$-alkylaminocarbonyle et di($C_1$-$C_8$-alkyl)aminocarbonyle, un groupe carbocyclique $C_3$-$C_{15}$, un groupe carbocyclique aromatique à membres $C_6$-$C_{15}$, un groupe hétérocyclique de 4 à 14 membres, cyano-$C_1$-$C_8$-alkyle, hydroxy-$C_1$-$C_8$-alkyle, $C_1$-$C_8$-haloalkyle, amino-$C_1$-$C_8$-alkyle, amino(hydroxy)$C_1$-$C_8$-alkyle et $C_1$-$C_8$-alkoxy substitué de façon optionnelle par aminocarbonyle.

**2.** Un composé de formule (I) selon la revendication 1, où le composé est de formule (Ia)

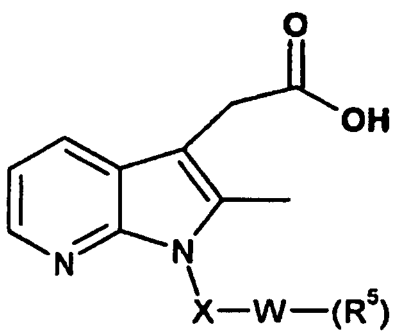

**(Ia)**

sous forme libre ou saline, où :

W est phényle ou indanyle ;
X est une liaison quand W est indanyle et, quand W est phényle, X est $C_2$-$C_8$-alkylène ou $C_1$-$C_8$-alkylèneoxy ;
$R^5$ n'est pas présent ou est un ou deux groupe(s) indépendamment sélectionné(s) parmi halogène, $C_1$-$C_8$-haloalkyle, $C_1$-$C_8$-alkylsulfonyle, -$SO_2N(R^{5a})R^{5b}$, où $R^{5a}$ et $R^{5b}$ sont indépendamment sélectionnés parmi $C_1$-$C_8$-alkyle et un groupe carbocyclique $C_3$-$C_{15}$ ou $R^{5a}$ et
$R^{5b}$ ensemble avec l'atome d'azote auquel ils sont attachés forment un groupe hétérocyclique de 4 à 14 membres.

**3.** Un composé selon la revendication 1 ou 2 ayant la formule

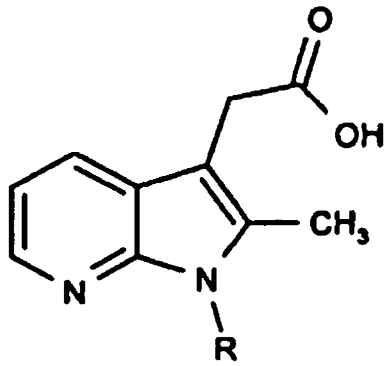

où R est (Example = Exemple)

| Example | R |
|---|---|
| 1 | 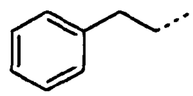 |
| 2 | 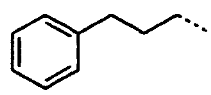 |
| 3 | 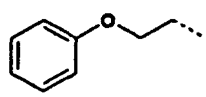 |
| 4 | 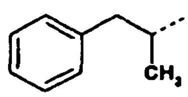 |
| 5 | 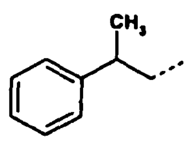 |
| 6 | 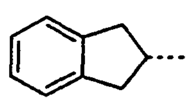 |
| 7 | 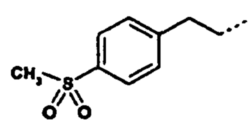 |
| 8 | 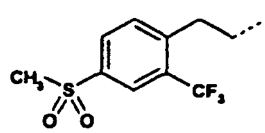 |

(suite)

| Example | R |
|---|---|
| 9 | 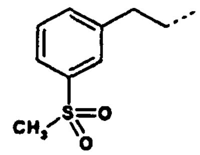 |
| 10 | 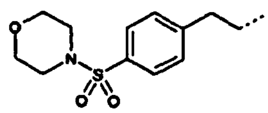 |
| 11 | 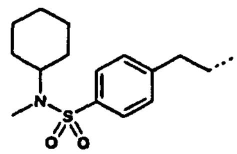 |
| 12 | 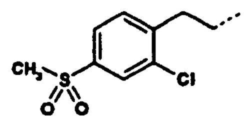 |
| 13 | 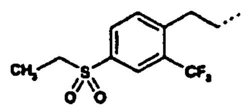 |
| 14 | 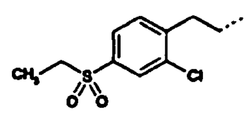 |
| 15 | 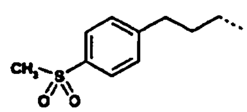 |
| 16 | 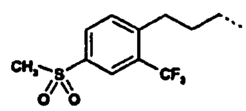 |
| 17 | 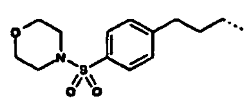 |
| 18 | 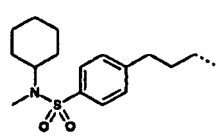 |
| 19 | 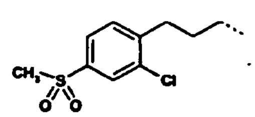 |
| 20 | 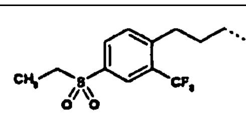 |

(suite)

| Example | R |
|---|---|
| 21 | 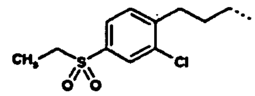 |
| 22 | 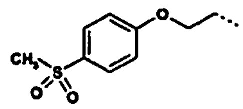 |
| 23 | 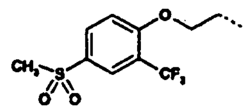 |
| 24 | 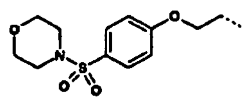 |
| 25 | 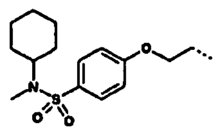 |
| 26 | 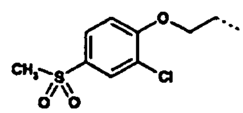 |
| 27 | 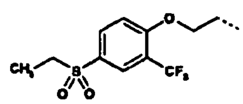 |
| 28 | 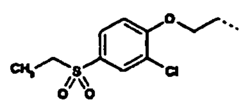 |
| 29 | 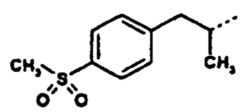 |
| 30 | 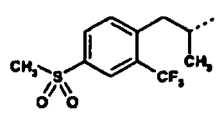 |
| 31 | 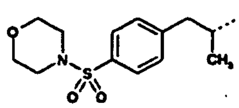 |
| 32 | 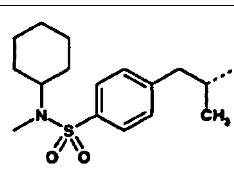 |

(suite)

| Example | R |
|---|---|
| 33 | 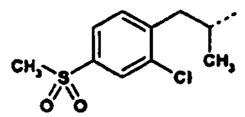 |
| 34 | 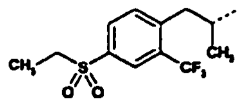 |
| 35 | 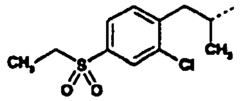 |
| 36 | 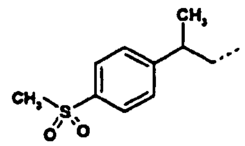 |
| 37 | 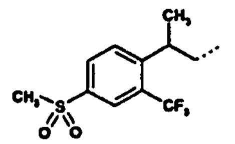 |
| 38 | 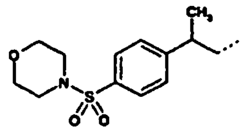 |
| 39 | 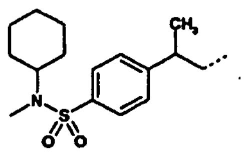 |
| 40 | 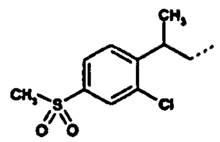 |
| 41 | 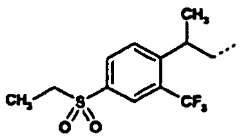 |
| 42 | 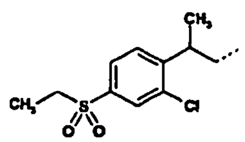 |
| 43 | 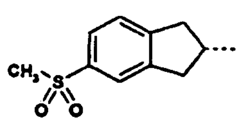 |

**4.** Un composé selon n'importe laquelle des revendications 1 à 3 pour une utilisation comme produit pharmaceutique.

**5.** Des compositions pharmaceutiques comportant un composé selon n'importe laquelle des revendications 1 à 3.

**6.** Un composé selon n'importe laquelle des revendications 1 à 3 pour le traitement de la douleur neuropathique.

**7.** Un composé selon n'importe laquelle des revendications 1 à 3 pour le traitement d'un état inflammatoire ou allergique, particulièrement une maladie inflammatoire ou obstructive des voies respiratoires.

**8.** Un procédé pour la préparation de composés de formule (I) comme défini dans la revendication 1, sous forme libre ou saline, qui comporte les étapes suivantes :

(i) (A) pour la préparation de composés de formule (I), où $R^6$ est H, attacher le groupe ester $-COOR^6$ dans un composé de formule (I),

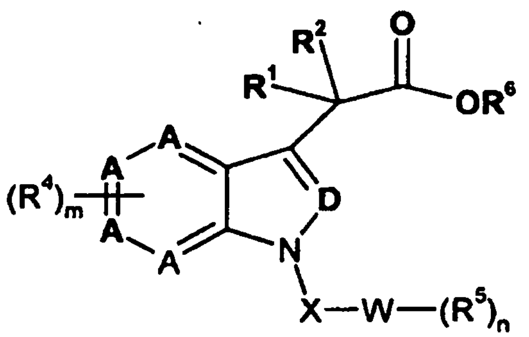

(I)

où $R^6$ est $C_1$-$C_8$-alkyle substitué de façon optionnelle par un groupe carbocyclique $C_3$-$C_{15}$ et $R^1$, $R^2$, $R^4$, $R^5$, A, D, W, X, m, et n sont comme définis auparavant ; ou
(B) pour la préparation de composés de formule (I), où $R^6$ est $C_1$-$C_8$-alkyle substitué de façon optionnelle par un groupe carbocyclique $C_3$-$C_{15}$, faire réagir un composé de formule (II)

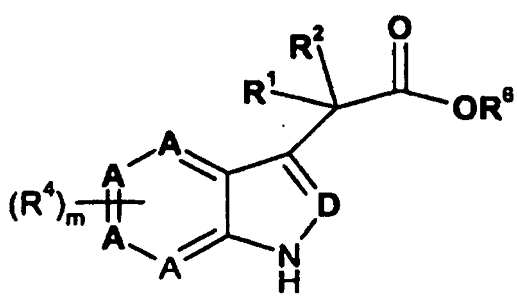

(II)

où
$R^6$ est $C_1$-$C_8$-alkyle substitué de façon optionnelle par un groupe carbocyclique $C_3$-$C_{15}$ et $R^1$, $R^2$, $R^4$, A, D, et m sont comme définis auparavant avec un composé de formule (III)
$G-X-W-(R^5)_n$ (III),
où
G est un groupe partant ;
$R^5$, W, X et n sont comme définis auparavant ; et
(ii) récupérer le composé résultant de formule (I) sous forme libre ou saline.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004069782 A
- US 3320268 A
- WO 05102338 A
- WO 0288167 A
- WO 0212266 A
- WO 02100879 A
- WO 0200679 A
- WO 03035668 A
- WO 03048181 A
- WO 03062259 A
- WO 03064445 A
- WO 03072592 A
- WO 0000531 A
- WO 0210143 A
- WO 03082280 A
- WO 03082787 A
- WO 03104195 A
- WO 04005229 A
- US 5451700 A
- WO 03104204 A
- WO 03104205 A
- WO 04000814 A
- WO 04000839 A
- WO 04005258 A
- WO 9818796 A
- WO 0339544 A
- EP 1052264 A
- EP 1241176 A
- EP 409595 A2
- WO 9417090 A
- WO 9602543 A
- WO 9602553 A
- WO 9828319 A
- WO 9924449 A
- WO 99124450 A
- WO 9924451 A
- WO 9938877 A
- WO 9941267 A
- WO 9967263 A
- WO 9967264 A
- WO 9967265 A
- WO 9967266 A
- WO 0023457 A
- WO 0077018 A
- WO 0078774 A
- WO 0123399 A
- WO 0127130 A
- WO 0127131 A
- WO 0160835 A
- WO 0194368 A
- WO 0200676 A
- WO 0222630 A
- WO 0296462 A
- WO 03086408 A
- WO 0242298 A
- WO 0075114 A
- WO 0416601 A
- WO 9964035 A
- WO 0142193 A
- WO 0183462 A
- WO 02066422 A
- WO 02070490 A
- WO 02076933 A
- WO 2004011416 A
- US 20020055651 A
- WO 0104118 A
- WO 0251841 A
- WO 0253564 A
- WO 0300840 A
- WO 0387094 A
- WO 0405285 A
- WO 0200652 A
- WO 0333495 A
- WO 0353966 A
- EP 0424021 A
- US 5171744 A
- US 3714357 A
- WO 2002026723 A
- WO 2003077907 A
- WO 2003007939 A
- WO 2002102775 A
- US 6166037 A
- WO 0066558 A
- WO 0066559 A


### Non-patent literature cited in the description


- **Szarka et al.** *J Immunol Methods,* 1997, vol. 202, 49-57
- **Renzi et al.** *Am Rev Respir Dis,* 1993, vol. 148, 932-939

- **Tsuyuki et al.** *J Clin Invest,* 1995, vol. 96, 2924-2931
- **Cernadas et al.** *Am J Respir Cell Mol Biol,* 1999, vol. 20, 1-8
- **Williams ; Galli.** *J Exp Med,* 2000, vol. 192, 455-462